# EUROPEAN PATENT SPECIFICATION

(11) **EP 0 749 472 B1**
(45) Date of publication and mention of the grant of the patent: **11.08.2004**
(21) Application number: 95913602.9
(22) Date of filing: 07.03.1995
(51) Int. Cl.: C12N 5/08

(54) **EXTRACORPOREAL CELL CULTURE AND TRANSPLANTATION KITS**
AUSRÜSTUNG FÜR DIE EXTRAKORPORALE ZELLKULTUR UND ZELLTRANSPLANTATION
CULTURE DE CELLULES EXTRA-CORPORELLE ET TROUSSES DE TRANSPLANTATION

(30) Priority: 07.03.1994 US 209502; 11.05.1994 US 243545; 06.03.1995 US 399404
(43) Date of publication of application: 27.12.1996
(73) Proprietor: IMMUNEX CORPORATION, Seattle Washington 98101 (US)
(72) Inventor: LYMAN, Stewart D., Seattle, WA 98103 (US)
(74) Representative: Bassil, Nicholas Charles
(86) International application number: PCT/US1995/002886
(87) International publication number: WO 1995/024469

(56) References cited:
- US-A- 5 199 942
- STEM CELLS (DAYT), NOV 1994, 12 (6) P626-37, UNITED STATES, XP000650431 LILL MC ET AL: "Production of functional myeloid cells from CD34-selected hematopoietic progenitor cells using a clinically relevant ex vivo expansion system."
- BLOOD, JUN 15 1993, 81 (12) P3463-73, UNITED STATES, XP000650417 MUENCH MO ET AL: "Bone marrow transplantation with interleukin-1 plus kit-ligand ex vivo expanded bone marrow accelerates hematopoietic reconstitution in mice without the loss of stem cell lineage and proliferative potential."
- CANCER RES;52(23):6576-82 1992, XP002024172 MUENCH MO ET AL: "Ex vivo differentiation therapy as a method of leukemic cell purging in murine bone marrow expansion cultures."
- BLOOD, Volume 81, Number 10, issued 15 May 1993, BRUGGER et al., "Ex Vivo Expansion of Enriched Peripheral Blood CD34+ Progenitor Cells by Stem Factor, Interleukin-1beta(IL-1beta)-IL-6, IL-3, Interferon-gamma, and Erythropoiten", pages 2579-2584.
- BIO/TECHNOLOGY, Volume 11, issued March 1993, PALSSON et al., "Expansion of Human Bone Marrow Progenitor Cells in a High Cell Density Continuous Perfusion System", pages 368-372.
- PROC. NATL. ACAD. SCI. U.S.A., Volume 88, issued April 1991, CULVER et al., "Lymphocytes as Cellular Vehicles for Gene Therapy in Mouse and Man", pages 3155-3159.
- LEUKEMIA AND LYMPHOMA, Volume 11, issued 1993, DURAND et al., "Long-Term Generation of Colony-Forming Cells (CFC) from CD34+ Human Umbilical Cord Blood Cells", pages 263-273.

## Description

### FIELD OF THE INVENTION

The invention pertains to cell selection and expansion technology and, in particular, to an extracorporeal cell culture and transplantation kit.

### BACKGROUND OF THE INVENTION

Cytoreductive therapies involve administration of ionizing radiation or chemical toxins that kill rapidly dividing cells. Side effects typically result from cytotoxic effects upon normal cells and can limit the use of cytoreductive therapies. A frequent side effect is myelosuppression, or damage to bone marrow cells that give rise to white and red blood cells and platelets. As a result of myelosuppression, patients develop cytopenia, or blood cell deficits, that increase risk of infection and bleeding disorders. Cytopenias increase morbidity, mortality, and lead to under-dosing in cancer treatment. On the other hand, high-dose chemotherapy is therapeutically beneficial because it can produce an increased frequency of objective response in patients with metastatic cancers, particularly breast cancer, when compared to standard dose therapy. This can result in extended disease-free remission for some even poor-prognosis patients. Nevertheless, high-dose chemotherapy is toxic and many resulting clinical complications are related to infections, bleeding disorders and other effects associated with prolonged periods of myelosuppression.

Many clinical investigators have manipulated cytoreductive therapy dosing regimens and schedules to increase dosing for cancer therapy, while limiting damage to bone marrow. An alternative method takes advantage of the fact that blood cells originate from hematopoietic stem cells that become committed to differentiate along certain lineages, i.e., erythroid, megakaryocytic, granulocytic, monocytic, and lymphocytic. Targeting these stem cells for cell separation therefore is of great interest in the treatment of cancer patients undergoing cytoreductive therapies. One therapeutic approach involves bone marrow or peripheral blood cell transplants in which bone marrow or circulating hematopoietic progenitor or stem cells are collected and proliferated in cell culture before cytoreductive therapy. The expanded cell population then can be reinfused following therapy to restore complete hematopoietic function. Optionally, a selecting step can be used to increase the relative numbers of hematopoietic progenitors in the collected explant. By reinfusing isolated stem or progenitor cells. reinfusion of other types of cells, including malignant cells, can be greatly minimized. In addition, during allogeneic transplantation, the number of T-cells transplanted can be greatly reduced by selecting only for stem or progenitor cells in order to minimize the risk of inducing Graft-versus-Host Disease (GvHD) in the patient.
A variety of cell selection techniques are known for identifying and separating hematopoietic stem or progenitor cells from a population of cells. Methods and materials for identifying and selecting such cell types are known. For example, monoclonal antibodies can be used to bind to a marker protein or surface antigen protein found on stem or progenitor cells. Such markers or cell surface antigens for hematopoietic stem cells include CD34, My-10, and Thy-1. In one method, antibodies are fixed to a surface, for example, glass beads, and contacted with a mixture of cells suspected of containing stem cells. This permits the antibodies to bind and secure the stem cells to the glass beads. Alternatively, the antibodies can be incubated with the cell mixture and the resulting combination contacted with a surface having an affinity for the antibody-cell complex. Undesired cells and cell matter are removed providing a relatively pure population of stem cells. Stem cells having the CD34 marker constitute only about 1% to 3% of the mononuclear cells in the bone marrow. The amount of CD34⁺ stem cells in the peripheral blood is approximately 10- to 100-fold less than in bone marrow. Thus, methods of increasing or expanding the numbers of isolated stem cells is desired to reduce the number of harvested stem cells needed from bone marrow of peripheral blood to provide rapid and full bone marrow recovery after ablative doses of radio- or chemotherapy.
Another method of cell selection involves the elimination of dividing cells with the use of certain antimetabolites. By combining cytokine stimulation with antimetabolite treatment, cell death can be induced in responding cells. Therefore, cells resistant to the proliferative effects of the cytokine(s) can be positively selected. See Berardi *et al*, Science, 267:104 (1995).
Bragger *et al*, *Blood* 81 (10) 1993, p2579-2584 relates to the effects of various growth factors on the *ex vivo* expansion of CD34⁺ progenitor cells for possible use in transplantation therapy. US 5,199,942 describes a method for autologous hematopoietic cell transplantation in a patient receiving cytoreductive therapy.
The use of expanded stem cells also allows for transplantation in situations in which an adequate number of stem cells cannot be harvested. A procedure termed extracorporeal stem cell culture and transplantation (ESCCAT), also known as *ex vivo* expansion, involves the removal of the autologous or allogenic stem cells, typically from the peripheral blood, bone marrow or umbilical cord blood, isolating the stem cells followed by the *in vitro* expansion of those cells. After expansion, the cells are infused into the patient.

### SUMMARY OF THE INVENTION

The invention is directed to cell selection and expansion technology and, in particular, to extracorporeal stem cell culture and transplantation (ESCCAT). More specifically, the invention is directed to an extracorporeal cell culture and transplantation kit that includes, but is not limited to, a cell binding protein for human hematopoietic stem or progenitor cells in a cell mixture collected from a human. The cell binding protein comprises at least one of flt3 receptor binding protein and a monoclonal antibody that binds to a cellular marker selected from the group consisting of: CD34, Thy-1 or My-10. The kit also includes a solid matrix or surface for isolating hematopoietic stem and progenitor cells from the cell mixture; a composition for expanding the isolated hematopoietic and progenitor cells comprising an effective amount of flt3-ligand and a growth factor selected from the group consisting of: GM-CSF, G-CSF, IL-1, IL-3, BL-6, TPO, EPO, SF and a GM-CSF/IL-3 fusion protein.
Alternatively, the cell binding protein and the solid matrix or surface are replaced with a growth factor for stimulating cellular division in non-hematopoietic stem and progenitor cells and an antimetabolite for inducing cell death in dividing cells. The antimetabolite may be selected from the group consisting of 5-fluorouracil and 4-hydroxycyclophosphamide and the growth factor is selected from the group consisting of SF and flt3-ligand.
Optionally, the kits according to the invention can comprise a container for containing the collected cell mixture, wherein the container is adapted to receive the collected cells and optionally, the selecting means.
The kit according to the invention provides a number of cellular expansion factors that are useful in ESCCAT for stimulating the proliferation of stem cells capable of self-renewal, and the proliferation and differentiation of lineage-committed progenitor cells. Such cellular growth factors include interleukins -1 and -3 (IL-1 and IL-3, respectively), granulocyte-macrophage colony stimulating factor (GM-CSF), molecular fusions of GM-CSF and IL-3 (PIXY321), flt3-ligand and granulocyte-colony stimulating factor (G-CSF). Other hematopoietic growth factors include stem-cell factor (SF) (also known as *c-kit* ligand, mast cell growth factor and steel factor), thrombopoietin (TPO), erythropoietin (EPO) and IL-6. These factors are useful for promoting the *in vitro* expansion of the isolated stem and progenitor cells.
The kits according to the invention are useful for selecting and expanding any cell population having the desired phenotype. For example, the kits find use in hematopoietic stem or progenitor cell expansion and transplantation, T cell or B cell expansion and transplantation, and gene therapy.

### DETAILED DESCRIPTION OF THE INVENTION

The invention is directed to cell selection and expansion technology and, in particular, to extracorporeal cell culture and transplantation. As described herein, the invention comprises an ESCCAT kit that comprises:
1) a cell binding protein for selecting human hematopoietic stem or progenitor cells in the cell mixture collected from a human;
2) a solid matrix or surface for isolating hematopoietic stem and progenitor cells from the cell mixture; and
3) a composition for expanding the isolated hematopoietic stem and progenitor cells comprising an effective amount of flt-3-ligand and a growth factor selected from the group consisting of: GM-CSF, G-CSF, IL-1, BL-3, IL-6, TPO, EPO, SF and a GM-CSF/IL-3 fusion protein.
The optional container is designed to initially receive the collected cells and optionally, the selecting means.
The cell mixture can be collected from a variety of sources. For selection of hematopoietic stem or progenitor cells, cells are typically collected from sources that include bone marrow, peripheral blood or umbilical cord blood.

### Definitions

The terms "stem cells" and "progenitor cells" refers to early-lineage cells that are pluripotential. The terms are herein used interchangeably, as is common in the art. The term "stem or progenitor cells" means either stem cells, progenitor cells, or a mixture of both stem and progenitor cells. As commonly used in the art, stem and progenitor cells typically are identifiable by the following cellular characteristics: CD34⁺, CD33⁻, CD38⁻, Thy-1⁺ and My-10⁺.
The term "flt3-L" refers to a genus of polypeptides that bind and complex independently with flt3 receptor found on progenitor and stem cells. Further encompassed by the term "flt3-L" are the proteins described in EP-A 627 487, which is incorporated herein by reference. The term "flt3-L" encompasses proteins having the amino acid sequence of 1 to 235 of SEQ. ID NO: 6 as shown in EP-A 627,487, as well as those proteins having a high degree of similarity or a high degree of identity therewith, and which proteins are biologically active and bind the flt3 receptor. In addition, the term refers to biologically active gene products of the DNA of SEQ. ID NO: 5 as shown in EP-A 627,487. Further encompassed by the term "flt3-L" are the membrane-bound proteins (which include an intracellular region, a membrane region, and an extracellular region), and soluble or truncated proteins which comprise primarily the extracellular portion of the protein, retain biological activity and are capable of being secreted. Specific examples of such soluble proteins are those comprising the sequence of amino acids 28-160 of SEQ ID NO:6 as shown in EP-A 627,487.

The term "IL-1" means either or both of the two forms, IL-1α and IL-1β (March et al., *Nature,* 315:641, 1985). Both IL-1α and IL-1β bind to IL-1 receptors (Type I and Type II). IL-1α is active in both precursor and mature forms whereas, IL-1β is active only in its mature form (March, et al. Id.). The term "IL-1" also refers to active fragments and analogs with altered amino acid sequences and derivatives, such as fusion proteins having an IL-1 component and IL-1 biological activity, see Mosley et al., *Proc. Natl. Acad. Sci.,* 84:4572 (1987).

The term "IL-3" refers to a genus of interleukin-3 polypeptides as described in U.S. Patent No. 5,108,910, incorporated herein by reference. Such polypeptides include analogs that have amino acid sequences that are substantially similar to the native human interleukin-3 amino acid sequences disclosed, for example, in EP publ. Nos. 275,598 and 282,185, each incorporated herein by reference. The term "IL-3" also includes analogs and alleles of IL-3 molecules that exhibit at least some of the biological activity in common with native human IL-3. Exemplary analogs of IL-3 are disclosed in EP Publ. No. 282,185. Other forms of IL-3 include human IL-3[Pro⁸Asp¹⁵Asp⁷⁰], human IL-3[Ser⁸Asp¹⁵Asp⁷⁰] and human IL-3[Ser⁸]. A DNA sequence encoding human IL-3 protein suitable for use in the invention is publicly available from the American Type Culture Collection (ATCC) under accession number ATCC 67747. The nomenclature used herein with respect to amino acid sequences in brackets designates which amino acids differ from the native human form. For example, human IL-3[Ser⁸Asp¹⁵Asp⁷⁰] refers to a human IL-3 protein in which amino acid 8 has been changed to a serine residue, amino acid 15 has been changed to an aspartic acid residue and the amino acid 70 has been changed to an aspartic acid residue.

The term "IL-6" refers to a genus of proteins as described in PCT Publ. WO 88/00206, EP 257406 and EP-A 331,640, each of which is incorporated herein by reference. IL-6 is identical to proteins termed "interferon-beta-2" (Zilberstein et al., *EMBO J*., 5:2529 (1986)) and the "26 kd protein inducible in human fibroblasts" (Haegeman et al., *Eur*. *J. Biochem.,* 159:625 (1986)). Such proteins include analogs that have an amino acid sequence that is substantially similar to the native human IL-6 amino acid sequences and which are biologically active in that they are capable of binding to a IL-6 receptor, transducing a biological signal initiated by binding IL-6 receptor, or cross-reacting with anti-IL-6 antibodies. Nucleotide sequences and deduced amino acid sequences of IL-6 are disclosed, for example in WO 88/00206. The term "IL-6" also includes analogs of native human IL-6 molecules sufficient to retain biological activity of native human IL-6.

As used herein, "GM-CSF" refers to a genus of proteins as described in U.S. Patent Nos. 5,108,910, and 5,229,496 each of which is incorporated herein by reference. Such proteins include analogs that have an amino acid sequence that is substantially similar to native human GM-CSF amino acid sequences (e.g., as publicly available ATCC 53157 or ATCC 39900), and which are biologically active in that they are capable of binding to a GM-CSF receptor, transducing a biological signal initiated by binding GM-CSF receptor, or cross-reacting with anti-GM-CSF antibodies. Amino acid sequences are disclosed, for example in Anderson, et al., *Proc*. *Natl. Acad. Sci., USA* 82:6250 (1985). Commercially available GM-CSF (sargramostim) is obtainable from Immunex Corp., Seattle, WA): The term "GM-CSF" also includes analogs of native human GM-CSF molecules sufficient to retain biological activity of native human GM-CSF. Exemplary analogs of GM-CSF include, for example, those described in EP Publ. No. 212914 and WO 89/03881, each of which is incorporated herein by reference. Other analogs of GM-CSF also may be used to construct fusion proteins with IL-3. A DNA sequence encoding a particularly preferred GM-CSF protein having potential glycosylation sites removed is publicly available from the ATCC under accession numbers ATCC 67231.

The term "GM-CSF/IL-3 fusion protein" means a C-terminal to N-terminal fusion of GM-CSF and IL-3. The fusion proteins are known and are described in U.S. Patent Nos. 5,199,942, 5,108,910 and 5.073,627, each of which is incorporated herein by reference. A preferred fusion protein is PIXY321 as described in US Patent No. 5,199,942.

The term "substantially similar" means variant amino acid sequence preferably is at least 80% identical to a native amino acid sequence, most preferably at least 90% identical. The percent identity may be determined, for example, by comparing sequence information using the GAP computer program, version 6.0 described by Devereux et al. (*Nucl*. *Acids Res.* 12:387, 1984) and available from the University of Wisconsin Genetics Computer Group (UWGCG). The GAP program utilizes the alignment method of Needleman and Wunsch (*J. Mol. Biol*. 48:443, 1970), as revised by Smith and Waterman. (*Adv*. *Appl. Math* 2:482, 1981). The preferred default parameters for the GAP program include: (1) a unary comparison matrix (containing a value of 1 for identities and 0 for non-identities) for nucleotides, and the weighted comparison matrix of Gribskov and Burgess, *Nucl. Acids Res.* 14:6745, 1986, as described by Schwartz and Dayhoff, eds., *Atlas of Protein* *Sequence and Structure,* National Biomedical Research Foundation, pp. 353-358, 1979; (2) a penalty of 3.0 for each gap and an additional 0.10 penalty for each symbol in each gap; and (3) no penalty for end gaps. Variants may comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known. Naturally occurring variants of the cellular expansion factors are also encompassed by the invention. Examples of such variants are proteins that result from alternate mRNA splicing events or from proteolytic cleavage of the native protein, wherein the native biological property is retained.

The term "purified or isolated" means that the purified or isolated material is substantially free of association with other cells, proteins or polypeptides, for example, as a purification product of recombinant host cell culture or as a purified extract.

The term "autologous transplantation" means a method in which cells having a desired phenotype are removed from a patient and re-administered to the same patient.

The term "allogeneic transplantation" means a method in which cells having a desired phenotype are removed from a human and administered to a different human. The term "syngeneic transplantation" means the cellular transplantation occurs between genetically identical humans.

The term "expansion" and "expanding" as used herein means enrichment of, or enriching, increasing, or providing an increase in, the numbers of the cells having the desired phenotype.

The term "ESCCAT" means a method comprising (1) collecting cells having a desired phenotype from a human; (2) expanding the cells *ex vivo* with a composition containing an effective amount of a cellular expansion growth factor to provide a cellular preparation comprising increased numbers of the desired cells; and (3) administering the cellular preparation to the patient in conjunction with or following cytoreductive therapy.

The term "effective amount" as used in conjunction with the cellular expansion factors, means that amount of expansion factor necessary to achieve the desired level of cell expansion. It will be readily apparent to a person of ordinary skill in the art that an effective amount of a particular cellular expansion factor depends on a variety of variables. Such variables include, the level of expansion desired, whether or not the factor is combined with another factor, and the types of cells to be expanded. Determinations of the effective amount are well within the skill in the art.

In a preferred embodiment. the cellular expansion growth factor is selected from the group consisting of a GM-CSF/IL-3 fusion protein, IL-1α, or flt3-ligand.

In an alternate embodiment, the extracorporeal cell culture and transplantation kit according to the invention comprises a composition comprising an effective amount of an expansion factor selected from the group consisting of: G-CSF, IL-3, IL-6, TPO, EPO and SF.

In addition, the kits of the invention are useful in gene therapy. Gene therapy involves administration of exogenous DNA-transfected cells to a host that are allowed to engraft. See e.g., Boggs, *International J*. *Cell Cloning,* 8:80-96, (1990); Kohn et. al., *Cancer Invest*., 7(2):179-192 (1989); Lehn, *Bone Marrow Transpl*., 5:287-293 (1990); and Verma, *Scientific American,* pp. 68-84 (1990). Since genetic transfer of the exogenous DNA to the cells occurs when the cells are dividing, the efficiency of such transfer can be greatly increased using the kits according to the invention. Using a composition comprising an effective amount of at least one cellular expansion factor selected from the group consisting of: GM-CSF, G-CSF, IL-1α, IL-3, IL-6, TPO, EPO, flt3-ligand, SF and a GM-CSF/IL-3 fusion protein, will facilitate the selected cells to more rapid proliferation or differentiation. Therefore, genetic uptake in the collected cells can be greatly enhanced. Generally, gene therapy methods are known in the art and include the steps of (a) culturing isolated stem cells in growth media comprising at least one cellular expansion factor selected from the group listed above; (b) transfecting the cultured cells from step (a) with the exogenous gene; and (c) administering the transfected cells to the mammal.

In cases where it is desirable to select, isolate and expand hematopoietic stem or progenitor cells, a preferred kit is one wherein the means for isolating the hematopoietic stem cells comprises at least one of a) flt3 receptor binding protein and b) a monoclonal antibody that binds to a cellular marker selected from the group consisting of: CD34, Thy-1 or My-10.

With regard to the particular aspects of the kits of the invention, the kits comprise means for selecting the cells having the desired phenotype from the collected cell mixture. Choosing suitable selection means will depend on the desired phenotype of the cell to be isolated. Hematopoietic stem cells are selectable by virtue of their physical characteristics, such as expressing the membrane-bound flt3 receptor, or having the following cellular markers: CD34, Thy-1 and My-10. Monoclonal antibodies that recognize any of these antigens have been described in U.S. Patent No. 4,714,680 (anti-My-10) incorporated herein by reference, anti-CD34 is commercially available from Becton Dickinson, Franklin Lakes, NJ), and anti-Thy-1 monoclonal antibodies can be readily generated using the methods described by Dalchau et al., *J*. *Exp*. *Med.* 149:576 (1979), incorporated herein by reference. A flt3 receptor binding protein also may be used, such as anti-flt3 monoclonal antibodies or the flt3-ligand and described in EP-A 627 487, which flt3-ligand is available from Immunex Corporation, Seattle, WA). The cell binding protein is brought into contact with the collected cell mixture and the combination is allowed to incubate for a period of time sufficient to permit the binding of the desired cell to the cell binding protein.

An alternative means of selecting the quiescent stem cells is to induce cell death in the dividing, more lineage-committed, cell types using an antimetabolite such as 5-fluorouracil (5-FU) or an alkylating agent such as 4-hydroxycyclophosphamide (4-HC). The non-quiescent cells are stimulated to proliferate and differentiate by the addition of growth factors that have little or no effect on the stem cells, causing the non-stem cells to proliferate and differentiate and making them more vulnerable to the cytotoxic effects of 5-FU or 4-HC. See Berardi et al., *Science,* 267:104 (1995), which is incorporated herein by reference.

Further included in the kits of the invention are means for isolating the selected cells that have the desired phenotype. Isolation of the cells can be performed by using, for example, affinity chromatography, antibody-coated magnetic beads, or antibodies fixed to a solid matrix, such as glass beads, flasks, etc. Antibodies that recognize a stem cell surface marker can be fused or conjugated to other chemical moieties such as biotin - which can be removed with an avidin or a streptavidin moiety secured to a solid support; fluorochromes useful in fluorescence activated cell sorting (FACS), or the like. Preferably, isolation is accomplished by an immunoaffinity column. Immunoaffinity columns can take any form, but usually comprise a packed bed reactor. The packed bed in these bioreactors is preferably made of a porous material having a substantially uniform coating of a substrate. The porous material, which provides a high surface area-to-volume ratio, allows for the cell mixture to flow over a large contact area while not impeding the flow of cells out of the bed. Typical substrates include avidin and streptavidin, while other conventional substrates can be used. The substrate should, either by its own properties, or by the addition of a chemical moiety, display high-affinity for a moiety found on the cell-binding protein such as a monoclonal antibody. The monoclonal antibodies recognize a cell surface antigen on the cells to be separated, and are typically further modified to present a biotin moiety. It is well-known that biotin has a high affinity for avidin, and the affinity of these substances thereby removably secures the monoclonal antibody to the surface of the packed bed. Such columns are well known in the art, see Berenson, et al., *J. Cell Biochem.,* 10D:239 (1986). The column is washed with a PBS solution to remove unbound material. Target cells can be released from the beads using conventional methods. Immunoaffinity columns of the type described above that utilize biotinylated anti-CD34 monoclonal antibodies secured to an avidin-coated packed bed are described for example, in PCT Publ. No. WO 93/08268. A variation of this method utilizes cell binding proteins, such as the monoclonal antibodies or flt3-ligand as described above, removably-secured to a fixed surface in the isolating means. The bound cell binding protein then is contacted with the collected cell mixture and allowed to incubate for a period of time sufficient to permit isolation of the desired cells.

Alternatively, the monoclonal antibodies that recognize the cell surface antigens can be labeled with a fluorescent label, e.g., chromophore or fluorophore, and separated by cell sorting according to the presence of absence or the amount of labeled product.

A further alternate embodiment of the isolating means is based on conventional magnetic separation methods and devices. An example of a method for coating a magnetic-intensifying gradient matrix for use in a separation apparatus is disclosed in U.S. Patent No. 5,385,707, incorporated herein by reference.

Further included in the kits of the invention are incubation means. Such means are adapted to receive and contain the isolated stem cells, a composition comprising an effective amount of a cellular expansion growth factor, and a cellular growth medium. The incubation means can be any device or apparatus that contains the isolated stem cells in contact with the expansion factor and the growth medium during the cellular expansion process. Suitable incubation means include, for example, bags, hollow fibers, glass bottles, multiple-well plates, or petri dishes. Many such incubation means are readily obtainable from a variety of commercial sources. Particularly preferred incubation means are sterile bags and hollow fibers.

Cellular expansion growth factors are also provided in the kits according to the invention. Such expansion factors include GM-CSF, G-CSF, IL-1, IL-3, IL-6, TPO, EPO, flt3-ligand, SF and a GM-CSF/IL-3 fusion protein. Preferred expansion factors are GM-CSF, flt3-ligand, IL-1α, and GM-CSF/IL-3 fusion proteins. The expansion factors are provided in the kits in the form of a composition that contains the factors. Examples of such compositions are those that comprise a recombinantly-produced, or otherwise purified, expansion factor in a conventional stabilizing formulation. Other compositions that can be included in the kit comprise conditioned media obtained from mammalian cells that contains an amount of expansion factor sufficient to expand the desired cells.

The kits according to the invention also comprise a cellular growth medium. A variety of growth media can be used, and the composition of such media can be readily determined by a person having ordinary skill in the art. Suitable growth media are solutions containing nutrients or metabolic additives, and include those that are serum-depleted or serum-based. Representative examples of growth media are RPMI, TC 199, Iscoves modified Dulbecco's medium (Iscove, et al., *F.J. Exp*. *Med.,* 147:923 (1978)), DMEM, Fischer's, alpha medium, NCTC, F-10, Leibovitz's L-15, MEM and McCoy's. Particular examples of nutrients that will be readily apparent to the skilled artisan include, serum albumin, transferrin, lipids, cholesterol, a reducing agent such as 2-mercaptoethanol or monothioglycerol, pyruvate, butyrate, and a glucocorticoid such as hydrocortisone 2-hemisuccinate. More particularly, the standard media includes an energy source, vitamins or other cell-supporting organic compounds, a buffer such as HEPES, Tris, that act to stabilize the pH of the media, various inorganic salts. Particular reference is made to PCT Publ. No. WO 95/00632, wherein a variety of serum-free cellular growth media is described, such disclosure is incorporated herein by reference.

As stated *supra*., the optional container for containing the cells collected from a human can be any sterile apparatus or device suitable for initially holding or containing the cell mixture. Preferably, the containing means is a sterile bag, having an opening for receiving the mixture of collected cells.

The kits of the invention can be used, for example, as follows in peripheral stem cell (PSC) or peripheral blood progenitor cell (PBPC) transplantation. Typically, PBPC and PSC transplantation is performed on patients whose bone marrow is unsuitable for collection due to, for example, marrow abnormality or malignant involvement. PBPC and PSC are collected using apheresis procedures known in the art. See, for example, Bishop et al., *Blood*, vol. 83, No. 2, pp. 610-616 (1994). Briefly, PBPC and PSC are collected using conventional devices, for example, a Haemonetics Model V50 apheresis device (Haemonetics, Braintree, MA). Four-hour collections are performed typically no more than five times weekly until approximately 6.5 x 10⁸ mononuclear cells (MNC)/kg patient are collected. The cells are suspended in standard media and then centrifuged to remove red blood cells and neutrophils. Cells located at the interface between the two phases (also known in the art as the buffy coat) are withdrawn and resuspended in HBSS. The suspended cells are predominantly mononuclear and a substantial portion of the cell mixture are early stem cells. The resulting stem cell suspension then is contacted with biotinylated anti-CD34 monoclonal antibodies. The contacting period is maintained for a sufficient time to allow substantial interaction between the anti-CD34 monoclonal antibodies and the CD34 antigens on the stem cell surface. Typically, times of at least one hour are sufficient. The cell suspension then is brought into contact with the isolating means provided in the kit. The isolating means can comprise a column packed with avidin-coated beads. Such columns are well known in the art, see Berenson, et al., *J. Cell Biochem*., 10D:239 (1986). The column is washed with a PBS solution to remove unbound material. Target stem cells can be released from the beads and from anti-GD34 monoclonal antibody using conventional methods. The stem cells obtained in this manner can be frozen in a controlled rate freezer (e.g., Cryo-Med. Mt. Clemons. MI), then stored in the vapor phase of liquid nitrogen. Ten percent dimethylsulfoxide can be used as a cryoprotectant. After all collections from the donor have been made, the stem cells are thawed and pooled into the incubating means. Aliquots containing stem cells, growth medium provided in the kit, such as McCoy's 5A medium, 0.3% agar, and at least one of the expansion factors provided in the kit: recombinant human GM-CSF, recombinant human flt3-L, and recombinant human GM-CSF/IL-3 fusion molecules (PIXY321) at concentrations of approximately 200 U/mL, are cultured and expanded in the incubating means provided in the kit, at 37 °C in 5% CO₂ in fully humidified air for 14 days. Optionally, human IL-1α may be added to the cultures. The most preferred combination of expansion factors comprises flt3-L plus either IL-3 or a GM-CSF/IL-3 fusion protein). The expanded stem cells then can be reinfused intravenously to the patient.

## Claims

1. An extracorporeal cell culture and transplantation kit, comprising:
(a) a cell binding protein for selecting human hematopoietic stem and progenitor cells in a cell mixture collected from a human, wherein the cell binding protein comprises at least one of a) flt3 receptor binding protein or b) a monoclonal antibody that binds to a cellular marker selected from the group consisting of: CD34, Thy-1 and My-10;
(b) a solid matrix or surface for isolating hematopoietic stem and progenitor cells from the cell mixture, and;
(c) a composition for expanding the isolated hematopoietic stem and progenitor cells comprising an effective amount of flt3-ligand and a growth factor, wherein the growth factor is selected from the group consisting of: GM-CSF, G-CSF, IL-1, IL-3, IL-6, TPO, EPO, SF and a GM-CSF/IL-3 fusion protein.

2. A kit according to claim 1, wherein the cell binding protein and the solid matrix or surface are replaced with a growth factor for stimulating cellular division in non-hematopoietic stem and progenitor cells and an antimetabolite for inducing cell death in dividing cells, wherein the antimetabolite is selected from the group consisting of 5-fluorouracil and 4-hydroxycyclophosphamide and the growth factor is selected from the group consisting of SF and flt3-ligand.

3. A kit according to claims 1 or 2, further comprising a container for containing the collected cell mixture.

4. A kit according to any of claims 1-3, further comprising an apparatus for containing, at a minimum, the isolated hematopoietic stem and progenitor cells and the composition for expanding the cells during incubation.

5. A kit according to any of claims 1-4, wherein the apparatus is selected from the group consisting of sterile bags, hollow fibres, glass bottles, multiple-well plates or petri dishes.

6. A kit according to any of claims 1-5, further comprising a cellular growth medium.

7. A kit according to any of claims 1-6, wherein the monoclonal antibody is labeled with a fluorescent label.

8. A kit according to any of claims 1-7, wherein the cell mixture is collected from a human source, wherein the human source is selected from the group consisting of bone marrow, peripheral blood and umbilical cord blood.

9. A kit according to any of claims 1-8, wherein the ft13 receptor binding protein and/or monoclonal antibody are fused or conjugated to biotin.

10. A kit according to any of claims 1 and 3-9, wherein the solid matrix or surface further comprises a substrate selected from the group consisting of avidin and/or streptavidin.

11. A kit according to any of claims 1 and 3-10, wherein the cell binding protein for selecting the hematopoietic stem and progenitor cells is fixed to the solid matrix or surface.

12. A kit according to any of claims 1 and 3-11, wherein the solid matrix or surface comprises an affinity chromatography column or antibody-coated magnetic beads.

13. A kit according to any of claims 1 and 3-12, wherein the flt3 receptor binding protein is selected from the group consisting of flt3-ligand or a monoclonal antibody directed against the flt3 receptor.

14. A kit according to any of claims 1 and 3-13, wherein the growth factor is a GM-CSF/IL-3 fusion protein.

15. A kit according to any of claims 1 and 3-13, wherein the growth factor is IL-1.

16. A kit according to any of claims 1 and 3-13, wherein the growth factor is GM-CSF.

17. A kit according to any of claims 1 and 3-13, wherein the growth factor is IL-3.

18. A kit according to any of claims 1 and 3-13, wherein the growth factor is G-CSF.

19. A kit according to any of claims 1 and 3-13, wherein the growth factor is IL-6.

20. A kit according to any of claims 1 and 3-13, wherein the growth factor is TPO.

21. A kit according to any of claims 1 and 3-13, wherein the growth factor is EPO.

22. A kit according to any of claims 1 and 3-13, wherein the growth factor is SF.

23. A kit according to any of claims 1-22 for use in peripheral stem or peripheral blood progenitor cell transplantation.

## Patentansprüche

1. Ausrüstung für die extrakorporale Zellkultur und Zelltransplantation, welche Folgendes umfasst:
(a) ein aus einem Menschen gesammeltes Zellbindungsprotein zum Auswählen humaner hämatopoietischer Stamm- und Vorläuferzellen in einer Zellmischung, wobei das Zellbindungsprotein mindestens entweder a) ein flt3-Rezeptor-Bindungsprotein oder b) einen monoklonalen Antikörper aufweist, welches/r an einen zellulären Marker ausgewählt aus der Gruppe bestehend aus CD34, Thy-1 und My-10 bindet;
(b) eine feste Matrix oder Oberfläche zum Isolieren hämatopoietischer Stamm- und Vorläuferzellen aus der Zellmischung, und;
(c) eine Zusammensetzung zum Expandieren der isolierten hämatopoietischer Stamm- und Vorläuferzellen, welche eine effektive Menge an flt3-Ligand und einen Wachstumsfaktor aufweist, wobei der Wachstumsfaktor aus der Gruppe bestehend aus: GM-CSF, G-CSF, IL-1, IL-3, IL-6, TPO, EPO, SF und einem GM-CSF/IL-3-Fusionsprotein ausgewählt ist.

2. Ausrüstung nach Anspruch 1, wobei das Zellbindungsprotein und die feste Matrix oder Oberfläche mit einem Wachstumsfaktor zum Stimulieren der Zellteilung in nichthämatopoietischen Stamm- und Vorläuferzellen und einem Antimetabolit zum Induzieren des Zelltodes in sich teilenden Zellen ersetzt ist, wobei der Antimetabolit aus der Gruppe bestehend aus 5-Fluorouracil und 4-Hydroxycyclophosphamid ausgewählt ist, und der Wachstumsfaktor aus der Gruppe bestehend aus SF und flt3-Ligand ausgewählt ist.

3. Ausrüstung nach Anspruch 1 oder 2, welche weiters einen Behälter zum Aufnehmen der entnommenen Zellmischung umfasst.

4. Ausrüstung nach einem der Ansprüche 1-3, welche weiters eine Vorrichtung zum Aufnehmen zumindest der isolierten hämatopoietischen Stamm- und Vorläuferzellen und der Zusammensetzung zum Expandieren der Zellen während der Inkubation umfasst.

5. Ausrüstung nach einem der Ansprüche 1-4, wobei die Vorrichtung aus der Gruppe bestehend aus sterilen Beuteln, Hohlfasern, Glasflaschen, Multiple-Well-Platten oder Petrischalen ausgewählt ist.

6. Ausrüstung nach einem der Ansprüche 1-5, welche weiters ein Zellwachstumsmedium umfasst.

7. Ausrüstung nach einem der Ansprüche 1-6, wobei der monoklonale Antikörper mit einer Fluoreszenzmarkierung versehen ist.

8. Ausrüstung nach einem der Ansprüche 1-7, wobei die Zellmischung aus einer humanen Quelle gesammelt ist, wobei die humane Quelle aus der Gruppe bestehend aus Knochenmark, peripherem Blut und Nabelschnurblut ausgewählt ist.

9. Ausrüstung nach einem der Ansprüche 1-8, wobei das flt3-Rezeptor-Bindungsprotein bzw. der monoklonale Antikörper mit Biotin verschmolzen oder konjugiert ist.

10. Ausrüstung nach einem der Ansprüche 1 und 3-9, wobei die feste Matrix oder Oberfläche weiters ein Substrat ausgewählt aus der Gruppe bestehend aus Avidin und/oder Streptavidin aufweist.

11. Ausrüstung nach einem der Ansprüche 1 und 3-10, wobei das Zellbindungsprotein zum Auswählen der hämatopoietischen Stamm- und Vorläuferzellen an der festen Matrix oder Oberfläche fixiert ist.

12. Ausrüstung nach einem der Ansprüche 1 und 3-11, wobei die feste Matrix oder Oberfläche eine Affinitätschromatographie-Säule oder Antikörper-beschichtete magnetische Beads aufweist.

13. Ausrüstung nach einem der Ansprüche 1 und 3-12, wobei das flt3-Rezeptor-Bindungsprotein aus der Gruppe bestehend aus flt3-Ligand oder einem monoklonalen Antikörper, welcher gegen den flt3-Rezeptor gerichtet ist, ausgewählt ist.

14. Ausrüstung nach einem der Ansprüche 1 und 3-13, wobei der Wachstumsfaktor ein GM-CSF/IL-3-Fusionsprotein ist.

15. Ausrüstung nach einem der Ansprüche 1 und 3-13, wobei der Wachstumsfaktor IL-1 ist.

16. Ausrüstung nach einem der Ansprüche 1 und 3-13, wobei der Wachstumsfaktor GM-CSF ist.

17. Ausrüstung nach einem der Ansprüche 1 und 3-13, wobei der Wachstumsfaktor IL-3 ist.

18. Ausrüstung nach einem der Ansprüche 1 und 3-13, wobei der Wachstumsfaktor G-CSF ist.

19. Ausrüstung nach einem der Ansprüche 1 und 3-13, wobei der Wachstumsfaktor IL-6 ist.

20. Ausrüstung nach einem der Ansprüche 1 und 3-13, wobei der Wachstumsfaktor TPO ist.

21. Ausrüstung nach einem der Ansprüche 1 und 3-13, wobei der Wachstumsfaktor EPO ist.

22. Ausrüstung nach einem der Ansprüche 1 und 3-13, wobei der Wachstumsfaktor SF ist.

23. Ausrüstung nach einem der Ansprüche 1-22 zur Verwendung bei der Transplantation peripherer Stamm- oder peripherer Blutvorläuferzellen.

## Revendications

1. Culture cellulaire extracorporelle et trousse de transplantation, comprenant :
(a) une protéine de liaison cellulaire pour la sélection de cellules souches et progénitrices hématopoïétiques humaines dans un mélange de cellules prélevé chez un homme, dans laquelle la protéine de liaison cellulaire comprend au moins un parmi a) la protéine de liaison au récepteur flt3 ou b) un anticorps monoclonal qui se lie à un marqueur cellulaire sélectionné parmi le groupe comprenant : le CD34, le Thy-1 et le My-10;
(b) une matrice ou une surface solide pour l'isolement de cellules souches et progénitrices hématopoïétiques à partir du mélange de cellules, et;
(c) une composition pour réaliser l'expansion des cellules souches et progénitrices hématopoïétiques isolées comprenant une quantité efficace de ligand flt3 et un facteur de croissance, dans laquelle le facteur de croissance est sélectionné parmi le groupe comprenant : le GM-CSF, le G-CSF, l'IL-1, l'IL-3, l'IL-6, la TPO, l'EPO, le SF et la protéine de fusion GM-CSF/IL-3.

2. Trousse suivant la revendication 1, dans laquelle la protéine de liaison cellulaire et la matrice ou surface solide sont remplacées par un facteur de croissance pour la stimulation d'une division cellulaire chez des cellules souches et progénitrices non hématopoïétiques et un anti-métabolite pour induire une mort cellulaire chez les cellules en division, dans laquelle l'antimétabolite est sélectionné parmi le groupe comprenant le 5-fluorouracile et le 4-hydroxycyclophosphamide et le facteur de croissance est sélectionné parmi le groupe comprenant le SF et le ligand flt3.

3. Trousse suivant la revendication 1 ou 2, comprenant en outre un récipient pour contenir le mélange prélevé de cellules.

4. Trousse suivant l'une quelconque des revendications 1-3, comprenant en outre un appareillage pour contenir, au minimum, les cellules souches et progénitrices hématopoïétiques isolées et la composition pour réaliser l'expansion des cellules pendant l'incubation.

5. Trousse suivant l'une quelconque des revendications 1-4, dans laquelle l'appareillage est sélectionné parmi le groupe comprenant des sacs stériles, des fibres creuses, des bouteilles de verre, des plaques multipuits ou des boîtes de Pétri.

6. Trousse suivant l'une quelconque des revendications 1-5, comprenant en outre un milieu de croissance cellulaire.

7. Trousse suivant l'une quelconque des revendications 1-6, dans laquelle l'anticorps monoclonal est marqué par un marqueur fluorescent.

8. Trousse suivant l'une quelconque des revendications 1-7, dans laquelle le mélange de cellules est prélevé à partir d'une source humaine, dans laquelle la source humaine est sélectionnée parmi le groupe comprenant la moelle osseuse, le sang périphérique et le sang de cordon ombilical.

9. Trousse suivant l'une quelconque des revendications 1-8, dans laquelle la protéine de liaison au récepteur flt3 et/ou l'anticorps monoclonal sont fusionnés ou conjugués à de la biotine.

10. Trousse suivant l'une quelconque des revendications 1 et 3-9, dans laquelle la matrice ou la surface solide comprend en outre un substrat sélectionné parmi le groupe comprenant de l'avidine et/ou de la streptavidine.

11. Trousse suivant l'une quelconque des revendications 1 et 3-10, dans laquelle la protéine de liaison cellulaire pour la sélection des cellules souches et progénitrices hématopoïétiques est fixée sur la matrice ou la surface solide.

12. Trousse suivant l'une quelconque des revendications 1 et 3-11, dans laquelle la matrice ou surface solide comprend une colonne de chromatographie d'affinité ou des billes magnétiques recouvertes d'anticorps.

13. Trousse suivant l'une quelconque des revendications 1 et 3-12, dans laquelle la protéine de liaison au récepteur flt3 est sélectionnée parmi le groupe comprenant le ligand flt3 ou un anticorps monoclonal dirigé contre le récepteur flt3.

14. Trousse suivant l'une quelconque des revendications 1 et 3-13, dans laquelle le facteur de croissance est une protéine de fusion GM-CSF/IL-3.

15. Trousse suivant l'une quelconque des revendications 1 à 3-13, dans laquelle le facteur de croissance est l'IL-1.

16. Trousse suivant l'une quelconque des revendications 1 et 3-13, dans laquelle le facteur de croissance est le GM-CSF.

17. Trousse suivant l'une quelconque des revendications 1 et 3-13, dans laquelle le facteur de croissance est l'IL-3.

18. Trousse suivant l'une quelconque des revendications 1 et 3-13, dans laquelle le facteur de croissance est le G-CSF.

19. Trousse suivant l'une quelconque des revendications 1 et 3-13, dans laquelle le facteur de croissance est l'IL-6.

20. Trousse suivant l'une quelconque des revendications 1 et 3-13, dans laquelle le facteur de croissance est la TPO.

21. Trousse suivant l'une quelconque des revendications 1 et 3-13, dans laquelle le facteur de croissance est l'EPO.

22. Trousse suivant l'une quelconque des revendications 1 et 3-13, dans laquelle le facteur de croissance est le SF.

23. Trousse suivant l'une quelconque des revendications 1-22, pour une utilisation dans la transplantation de cellules souches périphériques ou progénitrices sanguines périphériques.
